# EUROPEAN PATENT SPECIFICATION

(11) **EP 1 095 013 B1**
(45) Date of publication and mention of the grant of the patent: **18.06.2003**
(21) Application number: 99934696.8
(22) Date of filing: 14.07.1999
(51) Int. Cl.: C07C 251/48, A01N 37/50

(54) **PHENYL-METHOXYIMINO-ACETIC ACID DERIVATIVES AS PESTICIDES**
PHENYL-METHOXYIMINO-ESSIGSÄUREDERIVATE ALS PESTIZIDE
DERIVES DE L'ACIDE PHENYL-METHOXIMINO-ACETIQUE UTILISES COMME PESTICIDES

(30) Priority: 16.07.1998 CH 151898
(43) Date of publication of application: 02.05.2001
(73) Proprietor: Bayer CropScience AG, 40789 Monheim (DE)
(72) Inventor: SZCZEPANSKI, Henry, CH-4323 Wallbach (CH); ZELLER, Martin, CH-5400 Baden (CH); ZIEGLER, Hugo, CH-4108 Witterswil (CH)
(86) International application number: EP9904985
(87) International publication number: WO00003974

(56) References cited:
- WO-A-97/47592

## Description

The present invention relates to new phenyl-methoxyimino-acetic acid derivatives having microbicidal activity, a process for their preparation, agrochemical compositions containing these active ingredients, as well as the use thereof in the control and prevention of plant-pathogenic fungi in agriculture and in horticulture.

The new compounds fall within formula I, 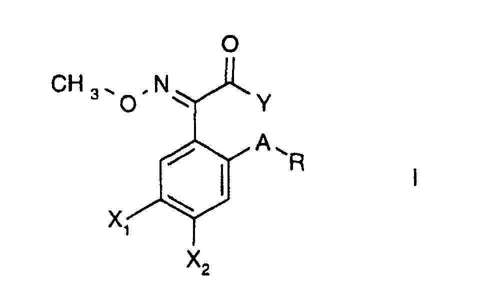 wherein:
A is a group CH₂O or CH₂ON=C(R₁);
X₁ and X₂ independently of one another, are C₁-C₄-alkyl;
Y is OH, O(C₁-C₄-alkyl), NH₂ or NHCH₃;
R₁ is C₁-C₄-alkyl, cyclopropyl, cyano, trifluoromethyl or C₁-C₄-alkoxy;
R is aryl, hetaryl or heterocyclyl, whereby the above-mentioned groups may be substituted by one or more identical or different substituents, selected from the group comprising halogen; C₁-C₆-alkyl; aryl which is optionally mono- to trisubstituted by identical or different substituents from C₁-C₄-alkyl, halogen, C₁-C₄-alkoxy, C₁-C₄-halogenalkyl or cyano;
C₁-C₆-alkoxy; halogen-C₁-C₆-alkoxy; aryloxy which is optionally mono- to trisubstituted by identical or different substituents from C₁-C₄-alkyl, halogen, C₁-C₄-alkoxy, C₁-C₄-halogenalkyl or cyano; halogen-C₁-C₆-alkyl; C₁-C₆-alkylthio; halogen-C₁-C₆-alkylthio;
C₁-C₆-alkylsulfinyl; halogen-C₁-C₆-alkylsulfinyl; C₁-C₆-alkylsulfonyl;
halogen-C₁-C₆-alkylsulfonyl; C₂-C₆-alkenyl; C₂-C₆-alkenyloxy; C₂-C₆-alkynyl; C₃-C₆-alkynyloxy; halogen-C₂-C₆-alkenyl; halogen-C₂-C₆-alkenyloxy; halogen-C₂-C₆-alkynyl; halogen-C₃-C₆-alkynyloxy; C₁-C₆-alkylcarbonyl; halogen-C₁-C₆-alkylcarbonyl;
C₁-C₆-alkoxycarbonyl; halogen-C₁-C₆-alkoxycarbonyl; C₁-C₆-alkylaminocarbonyl;
di-(C₁-C₆-alkyl)-aminocarbonyl, whereby the alkyl groups may be identical or different;
C₁-C₆-alkylaminothiocarbonyl; di-(C₁-C₆-alkyl)-aminothiocarbonyl, whereby the alkyl groups may be identical or different; C₁-C₆-alkylamino; di-(C₁-C₆-alkyl)-amino; NO₂; an unsubstituted C₁-C₄-alkylendioxy group or one which is mono- to tetrasubstituted by C₁-C₄-alkyl and/or halogen; CN; SF₅; OCN or C(=NOR₂)-Z-R₃;
R₂ and R₃ independently of one another, are hydrogen or C₁-C₆-alkyl;
Z is a direct bond, O, S, NH or N(C₁-C₆-alkyl).

Formula I is to include all possible isomeric forms and mixtures thereof, e.g. racemic mixtures and any [E/Z] mixtures.
Alkyl is either straight-chained, e.g. methyl, ethyl, propyl, butyl, pentyl or hexyl, or branched, e.g. isopropyl, isobutyl, sec.-butyl, tert.-butyl, isopentyl, neopentyl or isohexyl.
Alkenyl is either straight-chained, e.g. vinyl, 1-methylvinyl, allyl, 1-butenyl or 2-hexenyl, or branched, e.g. isopropenyl.
Alkynyl is either straight-chained, e.g. propargyl, 2-butinyl or 5-hexinyl, or branched, e.g. 2-ethinylpropyl or 2-propargylisopropyl.
Alkylenedioxy is -O(alkylene)O-.
Alkylene is either straight-chained, e.g. -CH₂CH₂-, -CH₂CH₂CH₂- or -CH₂CH₂CH₂CH₂-, or branched, e.g. -CH(CH₃)-, -CH(C₂H₅)-, -C(CH₃)₂-, -CH(CH₃)CH₂- or -CH(CH₃)CH(CH₃)-.
Halogen is fluorine, chlorine, bromine or iodine, preferably fluorine, chlorine or bromine. Halogenalkyl may contain identical or different halogen atoms.

Aryl signifies phenyl or naphthyl, preferably phenyl.
Heteroaryl signifies a cyclic aromatic group with 5 to 9 ring members in one or two rings, of which 1 to 3 members are hetero atoms selected from the group oxygen, sulphur and nitrogen. 1 to 2 benzene rings may be condensed on the heterocycle, the binding to the residual molecule taking place either through the hetero or the benzene moiety.
Examples are benzimidazolyl, benzisoxazolyl, benzisothiazolyl, benzocumarinyl, benzofuryl, benzothiadiazolyl, benzothiazolyl, benzothienyl, benzoxazolyl, benzoxdiazolyl, quinazolinyl, quinolyl, quinoxalinyl, carbazolyl, dihydrobenzofuryl, furyl, imidazolyl, indazolyl, indolyl, isoquinolinyl, isothiazolyl, isoxazolyl, methylenedioxyphenyl, ethylenedioxyphenyl, naphthyridinyl, oxazolyl, phenanthridinyl, phthalazinyl, pteridinyl, purinyl, pyrazinyl, pyrazolyl, pyridazinyl, pyrazolo[3,4-b]pyridyl, pyridyl, pyrimidinyl, pyrrolyl, tetrazolyl, oxadiazolyl, thiadiazolyl, thiazolyl, thienyl, triazinyl and triazolyl.

Preference is given to pyridyl, pyrazinyl, pyrimidinyl, thiazolyl, quinolinyl and thienyl.

Heterocyclyl signifies a 5- to 7-membered, non-aromatic ring with one to three hetero atoms selected from the group comprising N, O and S. Preference is given to non-aromatic 5- and 6-rings that have one nitrogen atom as a hetero atom and optionally one further hetero atom.
Piperidinyl, morpholinyl, pyrrolidinyl, pyrazolinyl, thiazolinyl and oxazolinyl are preferred.

Of the compounds of formula I, those groups are preferred, wherein:
(1) a) A is the group CH₂O; or
b) A is the group CH₂ON=C(R₁), wherein R₁ is CH₃; or
c) X₁ and X₂ are methyl; or
d) Y is OCH₃ or NHCH₃.

In addition, in combination with one of the above-mentioned groups (1)a to (1)d, the groups of compounds are preferred in which R has the following significances:
(2) phenyl which is unsubstituted or mono- to tri-substituted by identical or different substituents from halogen, C₁-C₆-alkyl, C₁-C₆-halogenalkyl, C₁-C₆-alkoxy, C₁-C₆-haloalkoxy, C₂-C₆-alkenyl, C₂-C₆-alkenyloxy, C₂-C₆-alkynyl, C₃-C₆-alkynyloxy, C₁-C₆-alkoxycarbonyl, cyano or OCN;
   especially phenyl which is mono- to disubstituted by identical or different substituents from halogen, C₁-C₂-alkyl, C₁-C₂-haloalkyl, C₁-C₂-alkoxy or C₁-C₂-haloalkoxy.
(3) phenyl which is substituted by
   C(=NOR₂ )-Z-R₃; wherein R₂ and R₃, independently of each other, signify hydrogen or C₁-C₄-alkyl and Z signifies a direct bond.
(4) pyridyl, pyrimidinyl, furyl, thienyl, oxazolyl, isoxazolyl, thiazolyl, isothiazolyl, imidazolyl, pyrazolyl, pyrazolinyl, which are unsubstituted or mono- to trisubstituted by identical or different substituents from halogen, cyano, nitro, aminocarbonyl, C₁-C₄-alkyl, C₁-C₄-halogenalkyl, C₁-C₄-alkylcarbonyl, C₁-C₄-alkylsulfonyl, C₁-C₆-alkylsulfoxyl, C₃-C₆-cycloalkyl, C₁-C₄-alkoxy, C₁-C₄-halogenalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₂-C₆-alkenyl, C(=NOR₂)-Z-R₃ or by aryl that is optionally mono- to trisubstituted by identical or different substituents from C₁-C₄-alkyl, halogen, C₁-C₄-alkoxy, C₁-C₄-halogenalkyl or cyano;
   especially
   pyridyl, pyrimidinyl, furyl, thienyl, oxazolyl, isoxazolyl, thiazolyl, isothiazolyl, imidazolyl, pyrazolyl, pyrazolinyl, which are unsubstituted or mono- to disubstituted by identical or different substituents from halogen, cyano, nitro, methyl, ethyl, halomethyl, haloethyl, methoxy, ethoxy, halomethoxy, haloethoxy, or by phenyl that is optionally mono- to disubstituted by identical or different substituents from C₁-C₂-alkyl, halogen, C₁-C₂-alkoxy, C₁-C₂-halogenalkyl or cyano.

Compounds of formula I may be produced as follows:
A) A compound of formula I is produced whereby a compound of the general formula II 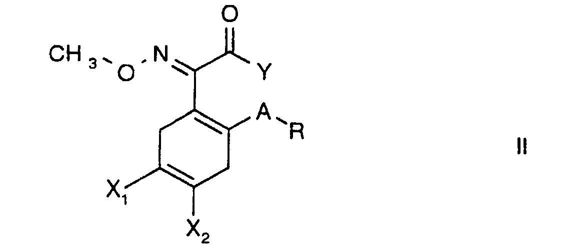 wherein A, R, X₁, X₂ and Y have the significances given for formula I, is reacted with an oxidation agent, such as a quinone, preferably chloranil or DDQ (2,3-dichloro-5,6-dicyano-1,4-benzoquinone), atmospheric oxygen, manganese dioxide, selenium dioxide, N,N,N',N'-tetramethyl ethylenediamine/base or triphenylmethanol in trifluoroacetic acid.
   The compounds of the general formula II are known (WO 97/47592 and WO 97/49672) or may be produced by the methods described therein.
B) A compound of formula I, wherein Y is OH, NH₂ or NHCH₃ and A, R, X₁ and X₂ have the significances given for formula I, are produced whereby a compound of formula la 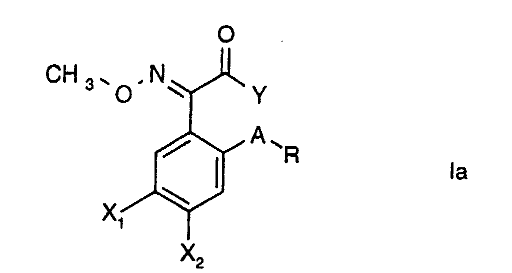 wherein Y signifies O(C₁-C₄ alkyl) and A, R, X₁ and X₂ have the significances given for formula I, is reacted with an aqueous acid or base, or with NH₃ or with NH₂CH₃.
C) A compound of formula I, wherein Y signifies O(C₁-C₄-alkyl) or NHCH₃, is produced whereby a compound of formula III, 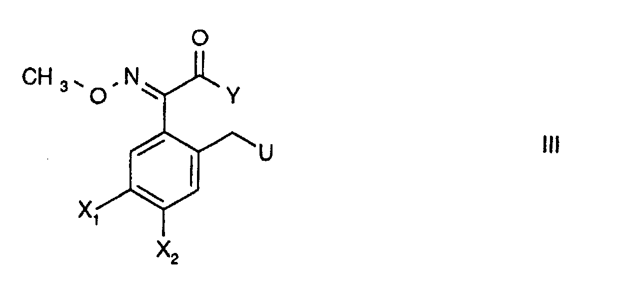 wherein U is a leaving group, for example chlorine, bromine, iodine, OH, mesyloxy, benzenesulphonyloxy or tosyloxy, preferably chlorine or bromine, and the remaining substituents have the above-mentioned significances, is reacted with an alcohol of the general formula IV, or with an oxime of the general formula V,

   R-OH IV

   R(R₁)C=NOH V

   wherein R and R₁ have the significances given for formula I.
D) A compound of formula I is produced whereby a compound of formula VI, 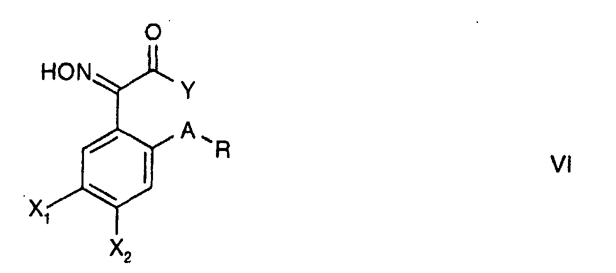 wherein the substituents have the significances given for formula I, is reacted with a methylation agent, e.g. methyl iodide or dimethyl sulphate.
   A compound of formula VI is produced, whereby either
   a) a compound of formula VII, 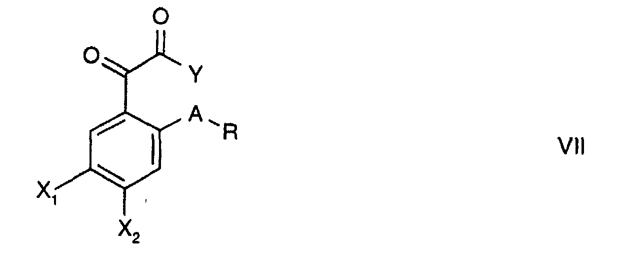
   wherein the substituents have the significances given for formula I, is reacted with hydroxylamine or with one of its salts, or
F) a compound of formula VIII, 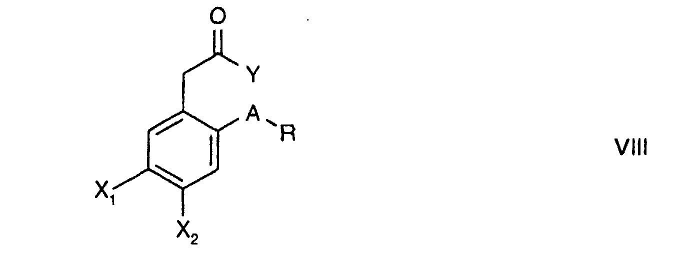 wherein the substituents have the significances given for formula I, is reacted with nitrous acid or with an alkyl nitrite.
G) A compound of formula I, wherein A is the group CH₂ON=CR₁ and the remaining substituents have the significances given for formula I, is produced whereby a compound of formula IX, 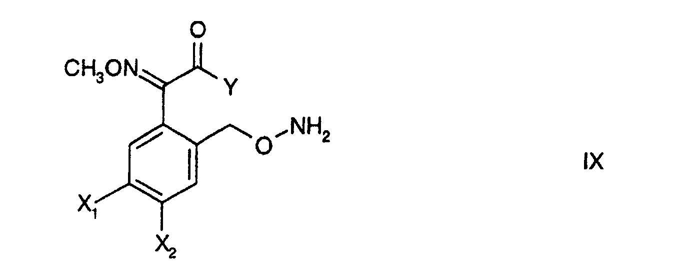 is reacted with a compound of formula R₁-CO-R, wherein the substituents have the significances given for formula I.
   All the above-described reactions are known *per se.*

The process for the production of the compounds of formula I, as well as the new intermediates of formulae III, VI, VII, VIII and IX likewise form objects of the invention.
The said intermediates may be produced by reacting the corresponding (analogous to formula II) 1,4-cyclohexadiene derivative with an oxidation agent, such as a quinone, preferably chloranil or DDQ (2,3-dichloro-5,6-dicyano-1,4-benzoquinone), atmospheric oxygen, manganese dioxide, selenium dioxide, N,N,N',N'-tetramethyl ethylenediamine/base or triphenylmethanol in trifluoroacetic acid.
The corresponding 1,4-cyclohexadiene derivatives are known (WO 97/47592 and WO 97/49672) or may be produced by the methods described therein.

The compounds of formula I are of preventive and/or curative merit as active ingredients for the control of plant pests and may be used in the agricultural sector and related fields The active ingredients of formula I according to the invention are notable for their good activity even at low concentrations, for their good plant tolerance and for their environmental acceptability. They possess very advantageous, especially systemic properties, and may be used for the protection of numerous cultivated plants. Using the active ingredients of formula I, pests appearing on plants or plant parts (fruits, flowers, foliage, stems, tubers, roots) of various crops can be checked or destroyed, whereby parts of the plant which grow later are also protected e.g. from phytopathogenic micro-organisms.
The compounds of formula I may also be be employed as a dressing for seeds (fruits, tubers, grain) and plant cuttings to protect against fungal infections, and to protect against phytopathogenic fungi appearing in the soil.

Compounds I are effective for example against the phytopathogenic fungi belonging to the following classes: Fungi imperfecti (e.g. *Botrytis, Pyricularia, Helminthosporium, Fusarium, Septoria, Cercospora* and *Alternaria*); *Basidiomycetes* (e.g. *Rhizoctonia, Hemileia, Puccinia*); *Ascomycetes* (e.g. *Venturia* and *Erysiphe, Podosphaera, Monilinia, Uncinula)* and *Oomycetes* (e.g. *Phytophthora, Pythium, Plasmopara*).

Target cultivations for the plant-protecting usage in the context of the invention are, for example, the following species of plant: cereals, (wheat, barley, rye, oats, rice, corn, sorghum and related species); beet (sugar beet and fodder beet); pomes, drupes and soft fruit (apples, pears, plums, peaches, almonds, cherries, strawberries, raspberries and blackberries); leguminous plants (beans, lentils, peas, soybean); oleaginous fruits (rape, mustard, poppy, olives, sunflowers, coconut, castor oil plants, cocoa beans, groundnuts); cucumber plants (squashes, cucumbers, melons); fibrous plants (cotton, flax, hemp, jute); citrus fruits (oranges, lemons, grapefruit, mandarins); vegetables (spinach, lettuce, asparagus, cabbages, carrots, onions, tomatoes, potatoes, paprika); *Lauraceae* (avocado, cinnamon, camphor); and plants such as tobacco, nuts, coffee, aubergines, sugar cane, tea, pepper, vines, hops, banana plants, natural rubber plants and ornamentals.

Further fields of application for the active ingredients according to the invention are the protection of stock and material, where the goods stored are protected against rotting and mildew.

Compounds I are used in this instance in unmodified form or preferably together with the excipients that are usual in formulation technology. To this end, they are suitably processed in known manner e.g. into emulsifiable concentrates, coatable pastes, directly sprayable or dilutable solutions, diluted emulsions, wettable powders, soluble powders, dusts or granules, e.g. by encapsulation in e.g. polymeric substances. As with the type of compositions, the methods of application, such as spraying, atomizing, dusting, scattering, coating or pouring, are selected in accordance with the intended objectives and the prevailing circumstances.
Suitable carriers and additives may be solid or liquid and are substances that are appropriate in formulation technology, for example natural or regenerated mineral substances, solvents, dispersants, wetting agents, tackifiers, thickeners, binding agents or fertilisers.

The compounds of formula I may be mixed with further active ingredients, e.g. fertilisers, trace element intermediates or other plant-protecting compositions, especially with further fungicides. Unexpected synergistic effects may thus occur.

Preferred mixture components are:
azoles, such as azaconazole, bitertanol, bromuconazole, cyproconazole, difenoconazole, diniconazole, epoxiconazole, fenbuconazole, fluquinconazole, flusilazole, flutriafol, hexaconazole, imazalil, imibenconazole, ipconazole, metconazole, myclobutanil, pefurazoate, penconazole, pyrifenox, prochloraz, propiconazole, tebuconazole, tetraconazole, triadimefon, triadimenol, triflumizole, triticonazole;
pyrimidinyl carbinols, such as ancymidol, fenarimol, nuarimol;
2-aminopyrimidines, such as bupirimate, dimethirimol, ethirimol;
morpholines, such as dodemorph, fenpropidin, fenpropimorph, spiroxamin, tridemorph; anilinopyrimidines, such as cyprodinil, mepanipyrim, pyrimethanil;
pyrroles, such as fenpiclonil, fludioxonil;
phenylamides, such as benalaxyl, furalaxyl, metalaxyl, R-metalaxyl, ofurace, oxadixyl;
benzimidazoles, such as benomyl, carbendazim, debacarb, fuberidazole, thiabendazole;
dicarboximides, such as chlozolinate, dichlozoline, iprodione, myclozoline, procymidone, vinclozolin;
carboxamides, such as carboxin, fenfuram, flutolanil, mepronil, oxycarboxin, thifluzamide;
guanidines, such as guazatine, dodine, iminoctadine;
strobilurines, such as azoxystrobin, kresoxim-methyl, SSF-126 (metominostrobin or
fenominostrobin; SSF-129 (α-methoximino-N-methyl-2-[(2,5-dimethylphenoxy)methyl]-benzeneacetamide), trifloxystrobin (2-[α-{[(α-methyl-3-trifluoromethyl-benzyl)imino]-oxy}-o-tolyl] -glyoxylic acid methylester-O-methyloxime);
dithiocarbamates, such as ferbam, mancozeb, maneb, metiram, propineb, thiram, zineb, ziram;
N-halomethylthio, such as captafol, captan, dichlofluanid, fluoromide, folpet, tolyfluanide; Cu compounds, such as Bordeaux mixture, copper hydroxide, copper oxychloride, copper sulphate, cuprous oxide, mancopper, oxine-copper;
nitrophenol derivatives, such as dinocap, nitrothal-isopropyl;
organo-P derivatives, such as edifenphos, iprobenphos, isoprothiolane, phosdiphen, pyrazophos, tolclofos-methyl;
miscellaneous, such as acibenzolar-S-methyl, anilazine, blasticidin-S, quinomethionat, chloroneb, chlorothalonil, cymoxanil, dichlone, diclomezine, dicloran, diethofencarb, dimethomorph, dithianon, etridiazole, famoxadone, fentin, ferimzone,fluazinam, flusulfamide, fenhexamid, fosetyl-aluminium, hymexazol, kasugamycin, methasulfocarb, pencycuron, phthalide, polyoxins, probenazole, propamocarb, pyroquilon, quinoxyfen, quintozene, sulphur, triazoxide, tricyclazole, triforine, validamycin.

One preferred method of applying an active ingredient of formula I or an agrochemical composition containing at least one of these active ingredients is application to the foliage (leaf application). The frequency and rate of application depend on the severity of infestation by the invader in question. However, the active ingredients I can also penetrate the plant through the roots via the soil (systemic action) by drenching the locus of the plants with a liquid preparation, or by applying the substances to the soil in solid form, for example in granular form (soil application). With paddy rice cultures, granules may be metered into the flooded paddy field. The compounds I may also be applied to seed grain for seed pretreatment (coating) by either drenching the grains or tubers in a liquid preparation of the active ingredient or coating them with a solid preparation.
The compositions are prepared in known manner, e.g. by intimately mixing and/or grinding the active ingredient with extenders, such as solvents, solid carriers and optionally surface-active compounds (surfactants).

The agrochemical compositions normally contain 0.1 to 99 percent by weight, especially 0.1 to 95 percent by weight, of active ingredient of formula I, 99.9 to 1 percent by weight, especially 99.8 to 5 percent by weight, of a solid or liquid additive and 0 to 25 percent by weight, especially 0.1 to 25 percent by weight, of a surfactant.

Favourable application rates generally lie between 1 g and 2 kg of active substance (AS) per hectare (ha), preferably between 10 g and 1 kg AS/ha, especially between 20 g and 600 g AS/ha.
For usage as a seed dressing, the dosages advantageously used are 10 mg to 1 g of active substance per kg seeds.

The compositions may also contain further additives, such as stabilisers, anti-foaming agents, viscosity regulators, binding agents or tackifiers, as well as fertilizers or other active ingredients, in order to achieve special effects.

### Preparation examples

### P-1) {4,5-dimethyl-2-[1-(3-trifluoromethyl-phenyl)-ethylidene-amino-oxymethyl]-phenyl}-methoxyimino-acetic acid methylester

A solution of 3.15 g of {4,5-dimethyl-2-[1-(3-trifluoromethyl-phenyl)-ethylidene-aminooxymethyl]-cyclohexa-1,4-dienyl}-methoxyimino-acetic acid methylester (as an E/Z-mixture in respect of the ethylidene-aminooxy double bond) in 40 ml of toluene is mixed with 2.0 g of 2,3-dichlor-5,6-dicyano-1,4-benzoquinone (DDQ), whereby a slight exothermic reaction is observed. After stirring for 4 hours at room temperature, the reaction mixture is filtered through Hyflo and the solvent is distilled off on a rotary evaporator. After purifying with diethyl ether/hexane (1:3) on silica gel, 2.6 g of the title compound are obtained as a yellowish oil.

### P-2) 2-{4,5-dimethyl-2-[1-(3-trifluoromethyl-phenyl)-ethylidene-amino-oxymethyl]-phenyl}-2-methoxyimino-N-methyl-acetamide

A solution of 2.1 g of the ester obtained in P-1 in 10 ml of ethanolic methylamine (8.03 molar) is stirred over night at room temperature. After distilling off the excess methylamine and the solvent, 2.0 g of the title compound are obtained in the form of a slightly yellow crystal powder having a melting range of 129-134°C (E/Z mixture).

### P-3) (4,5-dimethyl-2-o-tolyloxymethyl-phenyl)-methoxyimino-acetic acid methylester

A solution of 1.3 g of (4,5-dimethyl-2-o-tolyloxymethyl-cyclohexa-1,4-dienyl)-methoxyiminoacetic acid methylester in 15 ml of toluene is mixed with 1.1 g of DDQ. After stirring for 5 hours at room temperature, the reaction mixture is purified using toluene/hexane/diisopropylether (10:10:3) on silica gel. After stirring in hexane, the title compound is obtained in the form of white crystals having the melting point 82-84°C.

The compounds of the following tables may be produced in analogous manner.

### Table 1

Compounds of the general formula I.1, in which Y signifies methoxy and R corresponds in each case to one line of Table A.

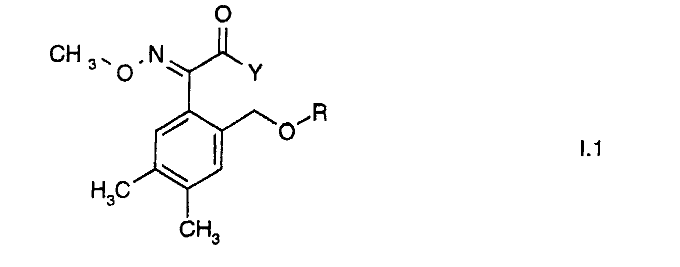

### Table 2

Compounds of the general formula I.1, in which Y signifies ethoxy and R corresponds in each case to one line of Table A.

### Table 3

Compounds of the general formula I.1, in which Y signifies NHCH₃ and R corresponds in each case to one line of Table A.

### Table 4

Compounds of the general formula 1.2, in which R₁ signifies methyl and Y signifies methoxy and R corresponds in each case to one line of Table A.

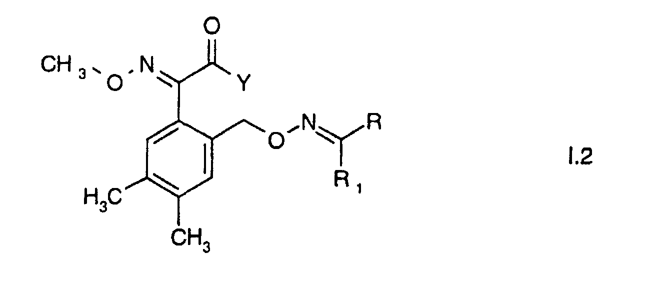

### Table 5

Compounds of the general formula 1.2, in which R₁ signifies methyl and Y signifies ethoxy and R corresponds in each case to one line of Table A.

### Table 6

Compounds of the general formula 1.2, in which R₁ signifies methyl and Y signifies NHCH₃ and R corresponds in each case to one line of Table A.

### Table 7

Compounds of the general formula 1.2, in which R₁ signifies ethyl and Y signifies methoxy and R corresponds in each case to one line of Table A.

### Table 8

Compounds of the general formula 1.2, in which R₁ signifies ethyl and Y signifies ethoxy and R corresponds in each case to one line of Table A.

### Table 9

Compounds of the general formula 1.2, in which R₁ signifies ethyl and Y signifies NHCH₃ and R corresponds in each case to one line of Table A.

### Table 10

Compounds of the general formula 1.2, in which R₁ signifies cyclopropyl and Y signifies methoxy and R corresponds in each case to one line of Table A.

### Table 11

Compounds of the general formula 1.2, in which R₁ signifies cyclopropyl and Y signifies ethoxy and R corresponds in each case to one line of Table A.

### Table 12

Compounds of the general formula 1.2, in which R₁ signifies cyclopropyl and Y signifies NHCH₃ and R corresponds in each case to one line of Table A.

### Table 13

Compounds of the general formula 1.2, in which R₁ signifies methoxy and Y signifies methoxy and R corresponds in each case to one line of Table A.

### Table 14

Compounds of the general formula 1.2, in which R₁ signifies methoxy and Y signifies ethoxy and R corresponds in each case to one line of Table A.

### Table 15

Compounds of the general formula 1.2, in which R₁ signifies methoxy and Y signifies NHCH₃ and R corresponds in each case to one line of Table A.

### Table 16

Compounds of the general formula 1.2, in which R₁ signifies cyano and Y signifies methoxy and R corresponds in each case to one line of Table A.

### Table 17

Compounds of the general formula 1.2, in which R₁ signifies cyano and Y signifies ethoxy and R corresponds in each case to one line of Table A.

### Table 18

Compounds of the general formula 1.2, in which R₁ signifies cyano and Y signifies NHCH₃ and R corresponds in each case to one line of Table A.

### Table 19

Compounds of the general formula 1.2, in which R₁ signifies trifluoromethyl and Y signifies methoxy and R corresponds in each case to one line of Table A.

### Table 20

Compounds of the general formula 1.2, in which R₁ signifies trifluoromethyl and Y signifies ethoxy and R corresponds in each case to one line of Table A.

### Table 21

Compounds of the general formula 1.2, in which R₁ signifies trifluoromethyl and Y signifies NHCH₃ and R corresponds in each case to one line of Table A.

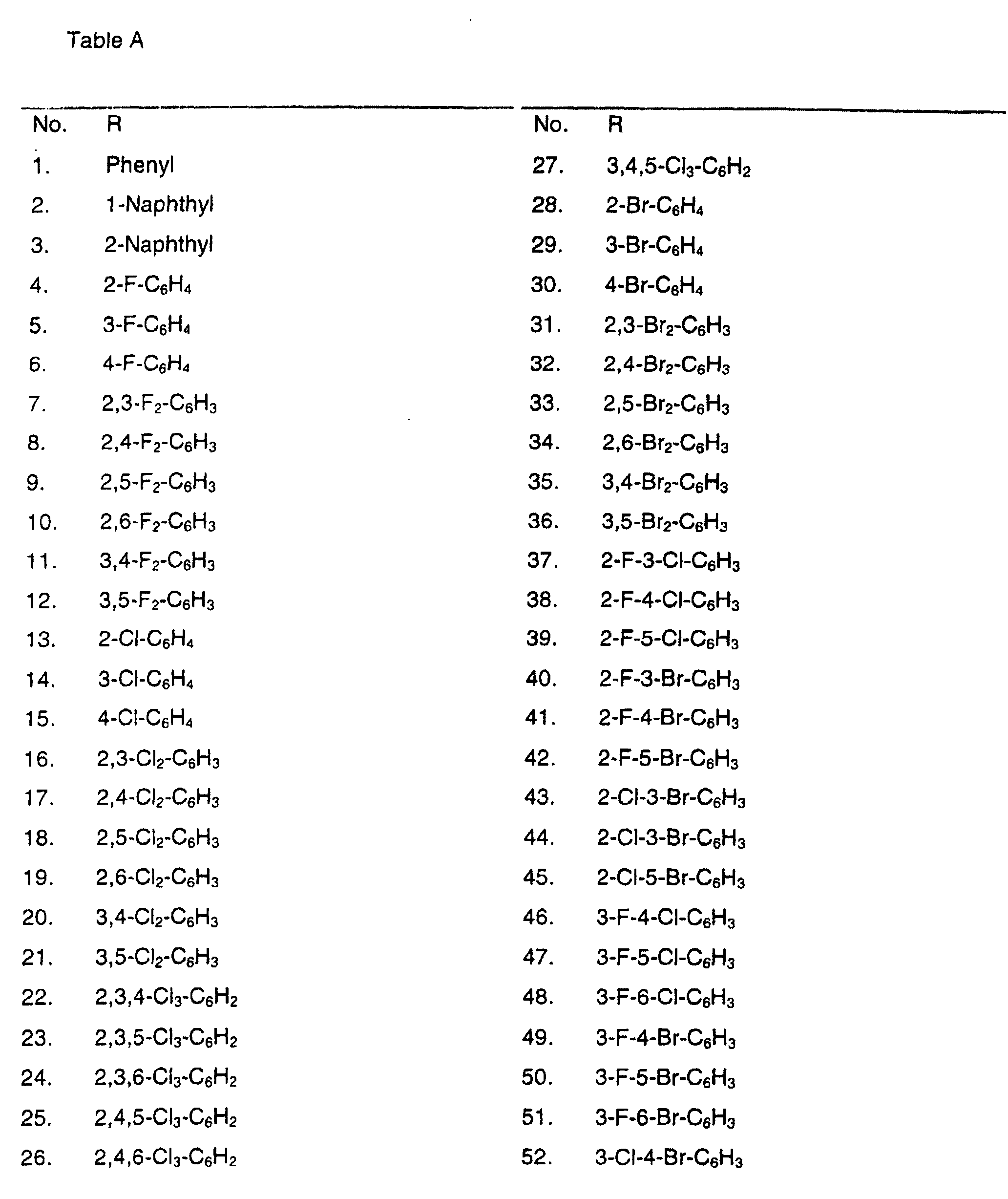

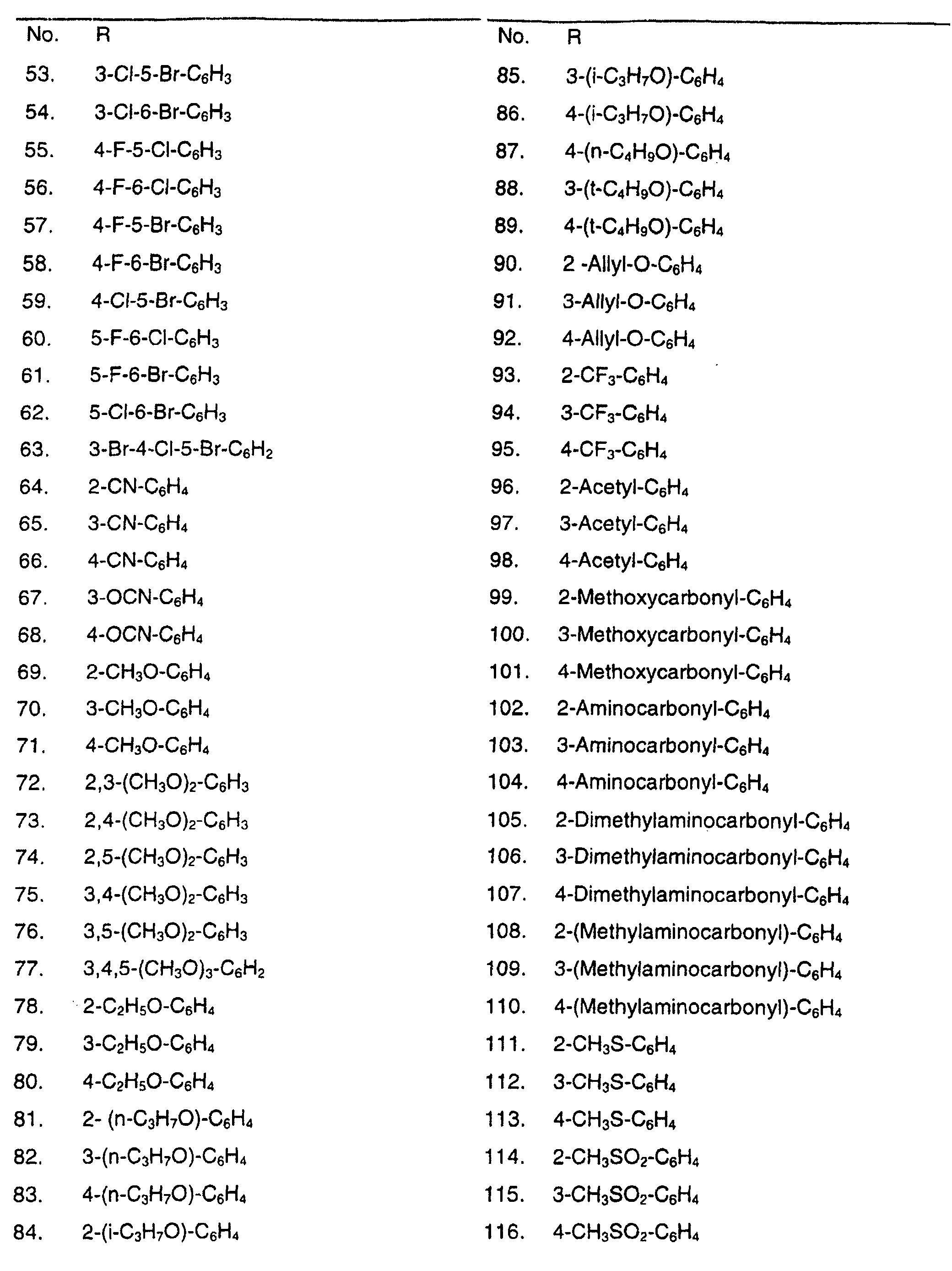

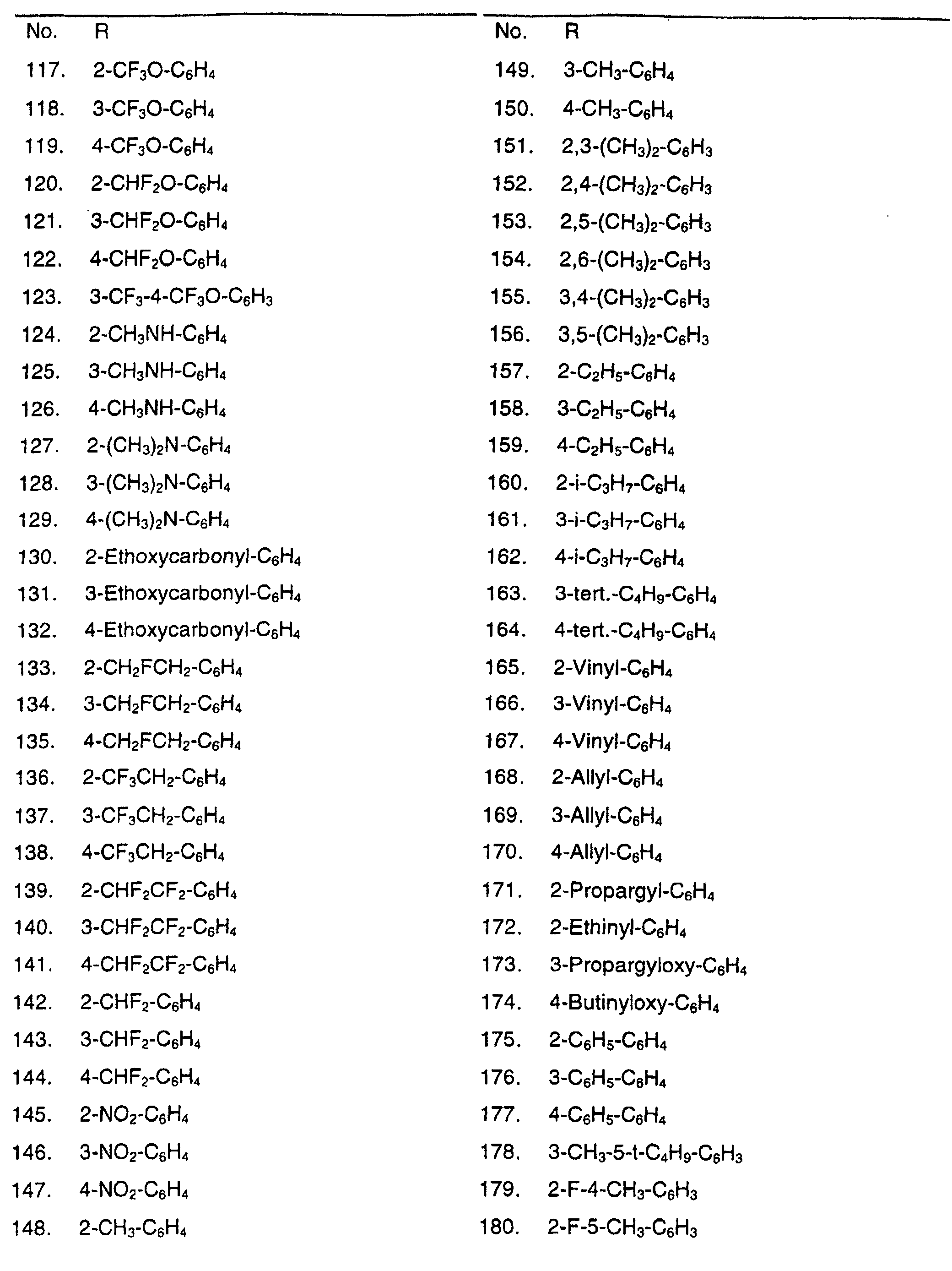

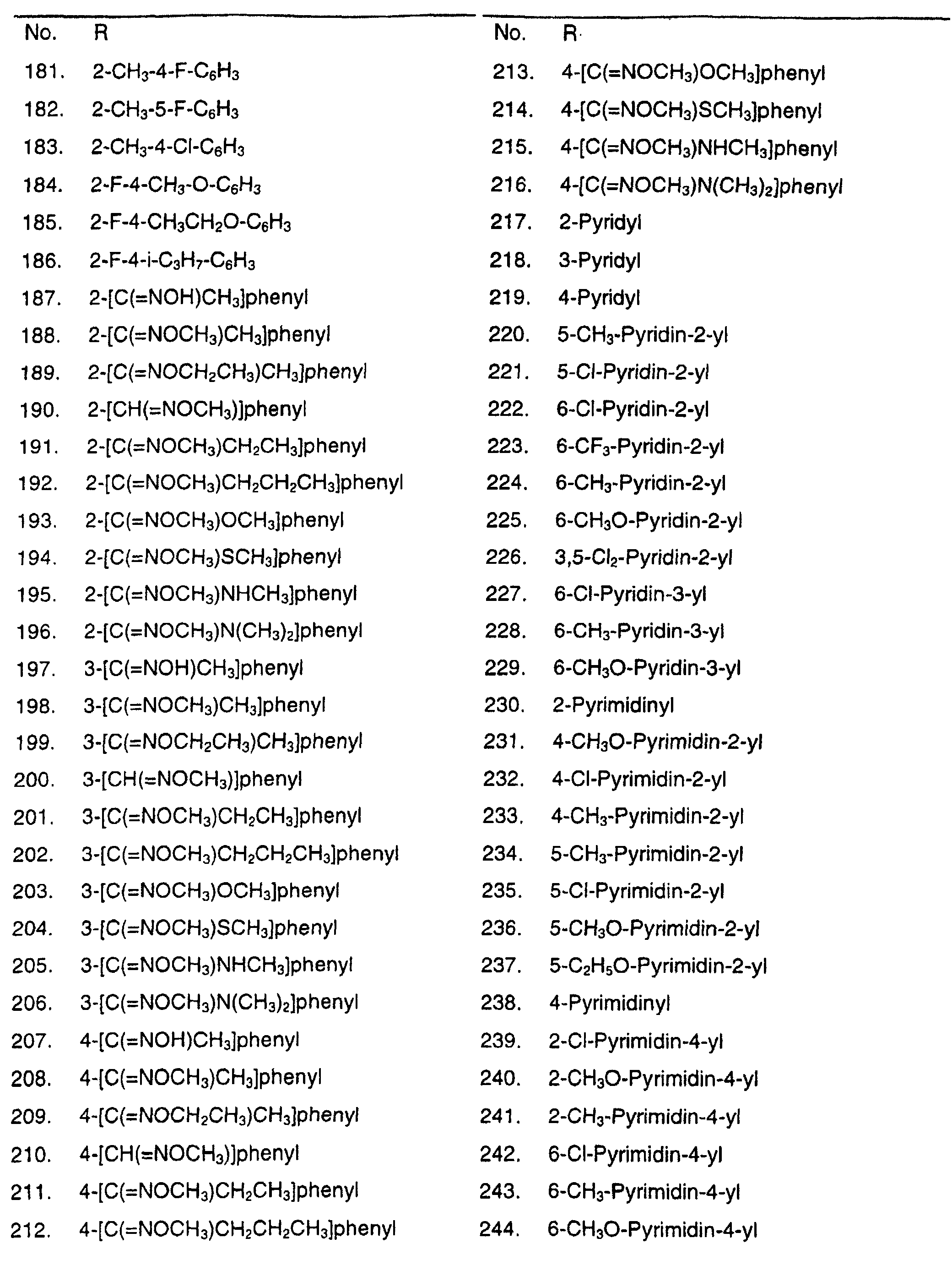

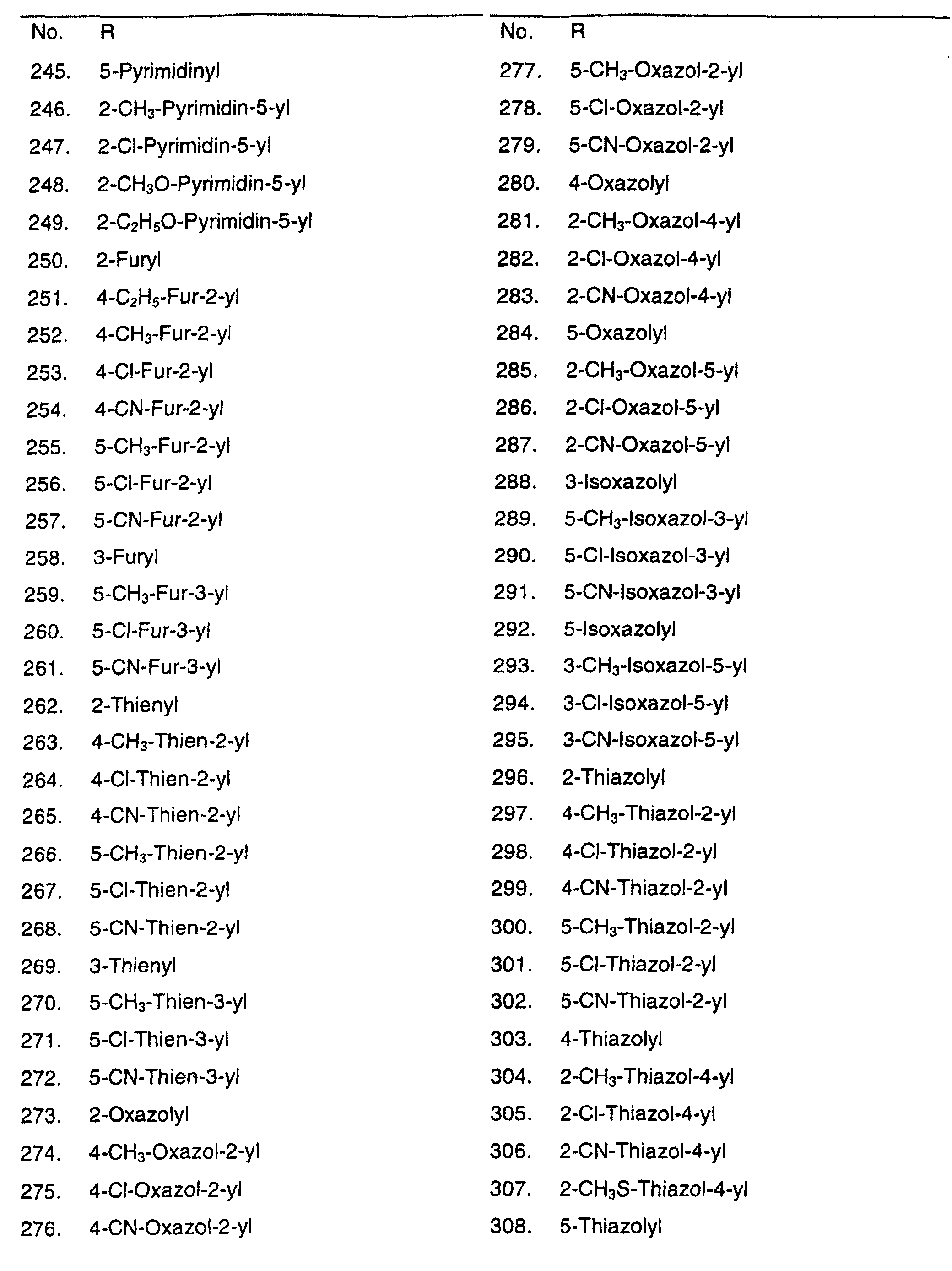

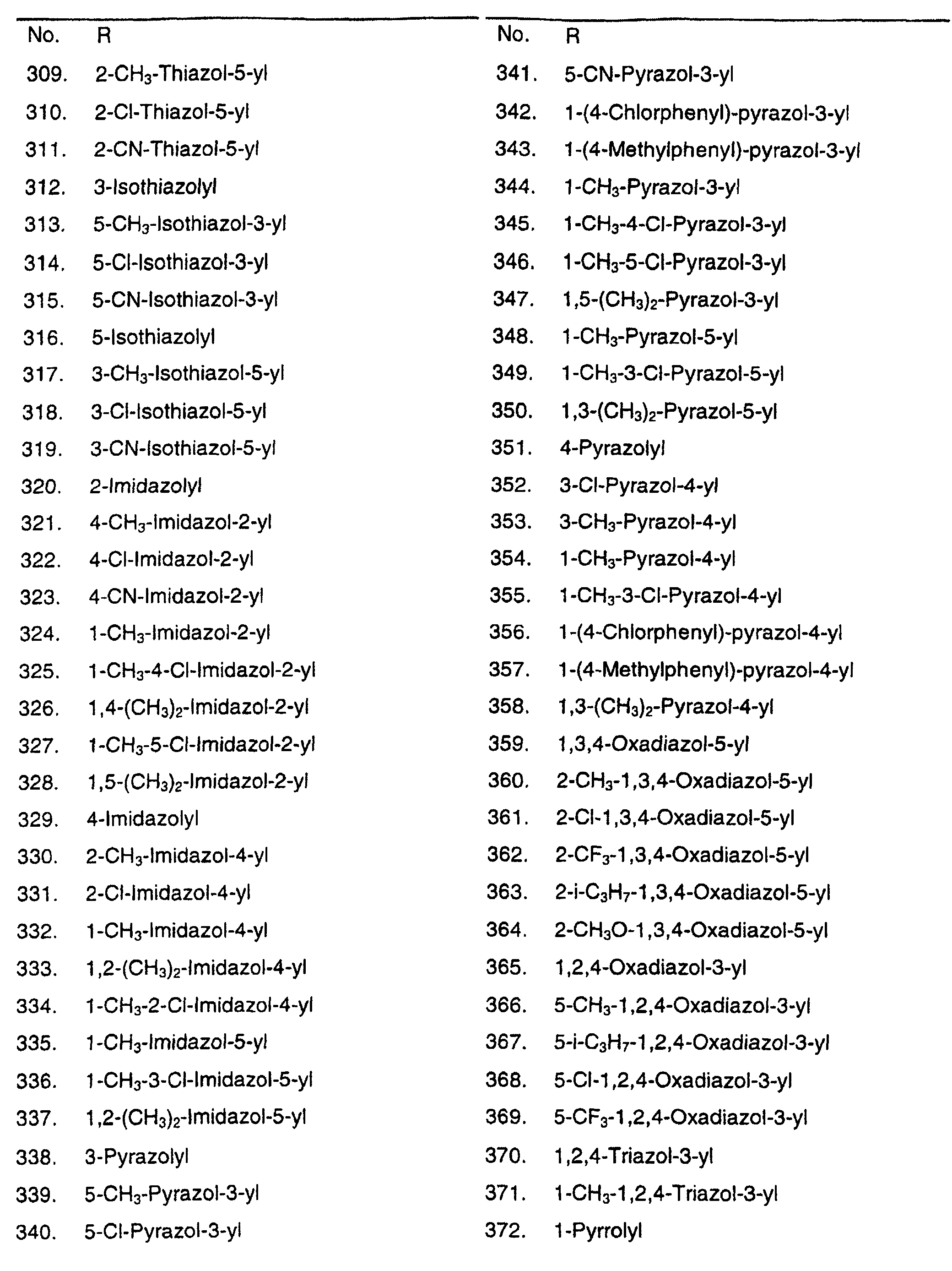

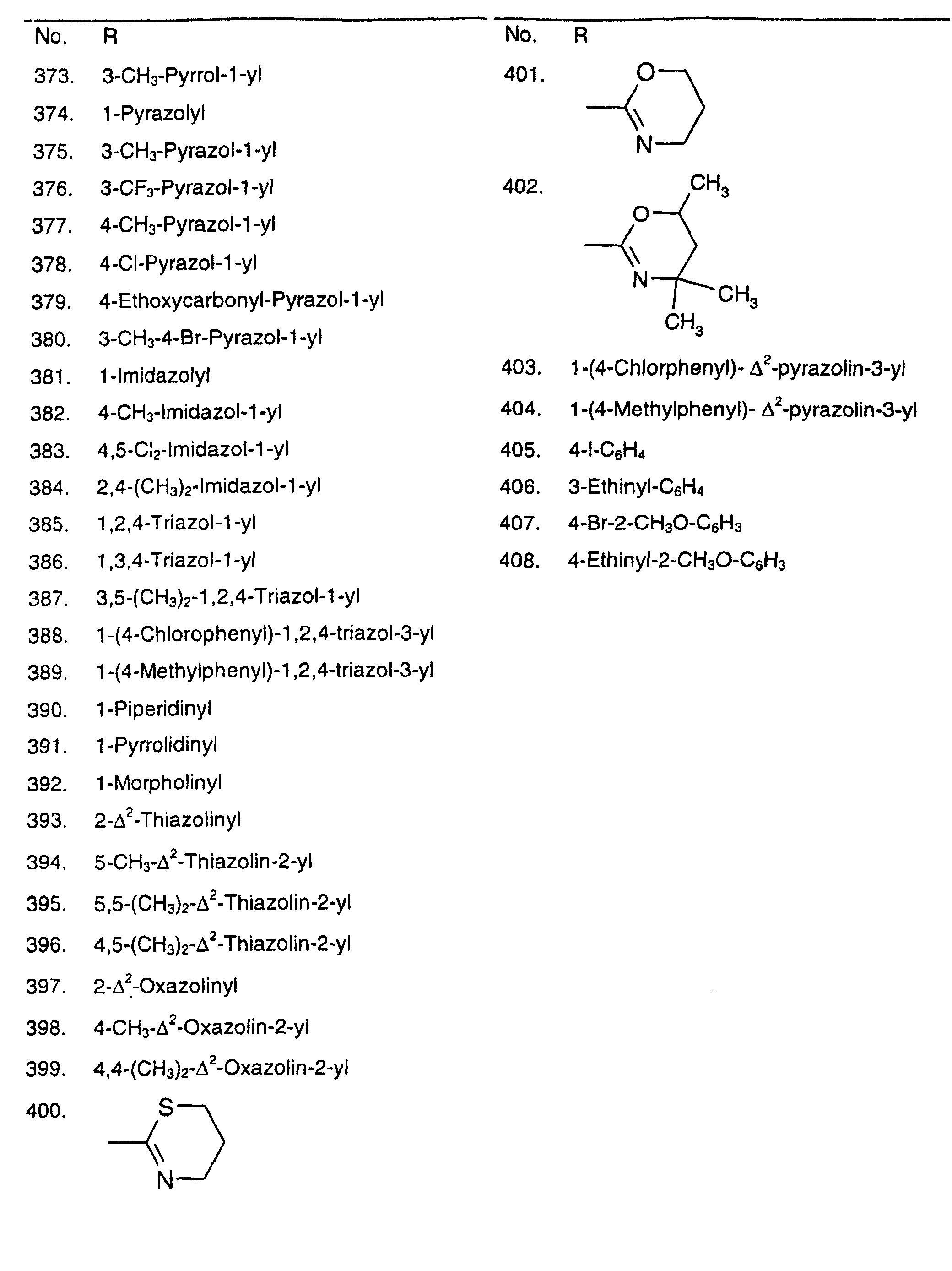

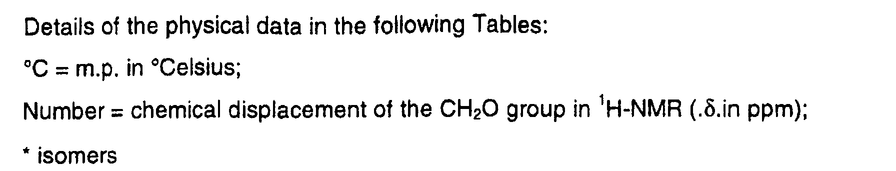

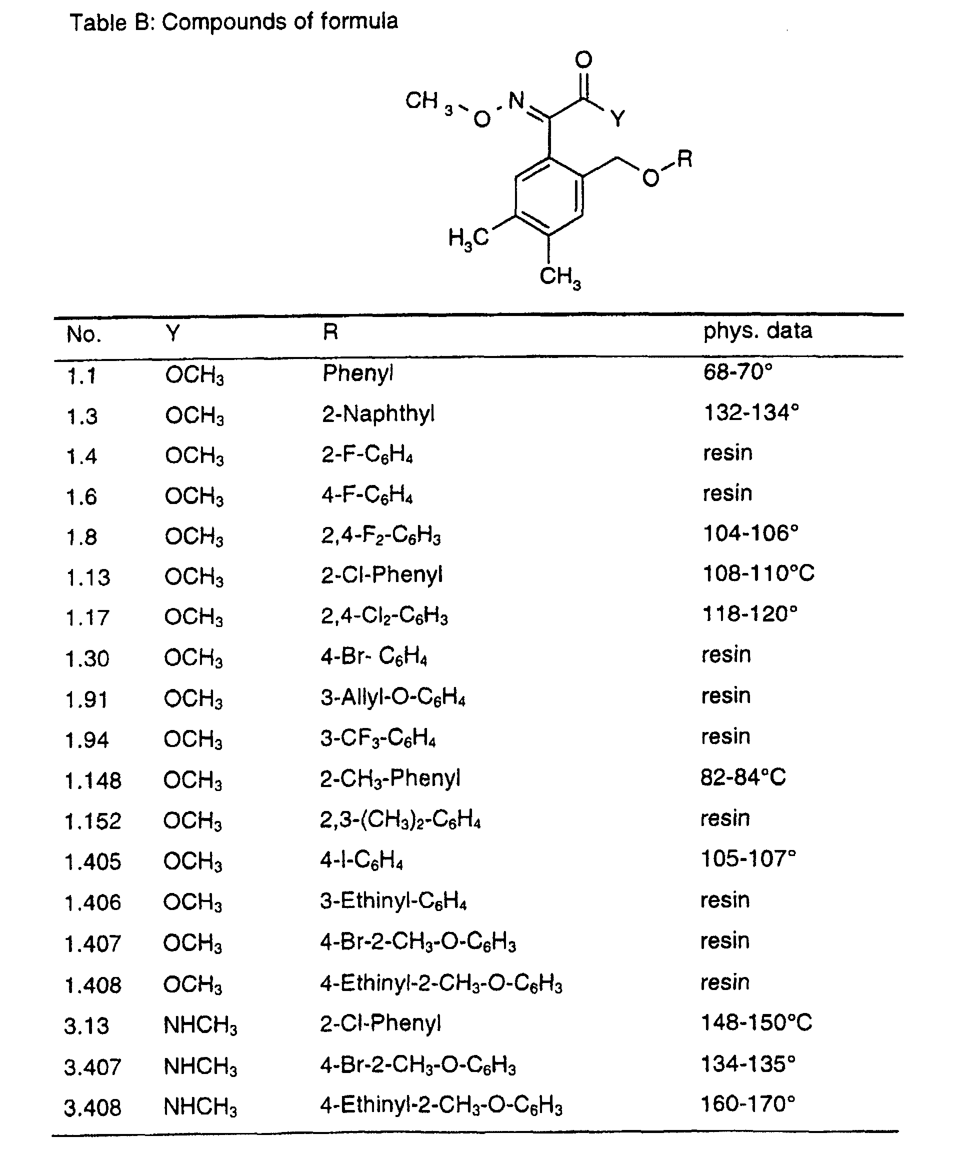

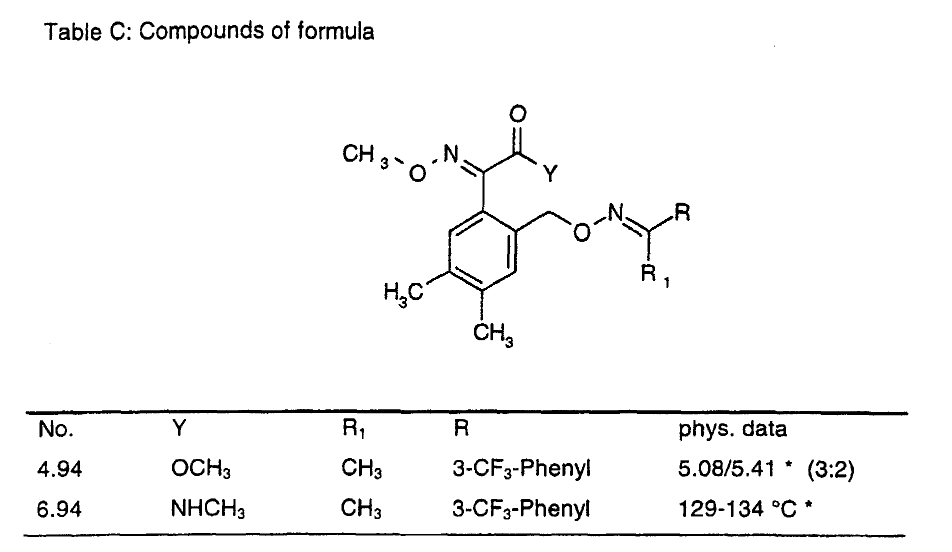

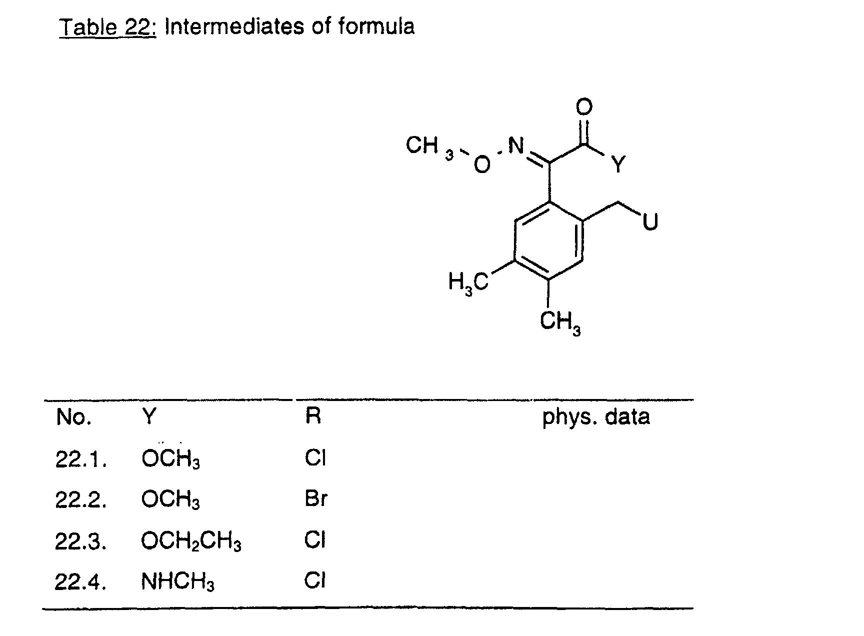

Formulations may be prepared analogously to those described for example in WO 97/33890.

### Biological Examples

In the following patho-systems, compounds from the tables display good activity.

### Example B-1: Activity against Puccinia graminis on wheat

### a) Residual protective action

6 days after planting, wheat plants are sprayed to drip point with an aqueous spray mixture prepared from a wettable powder of the active ingredient (0.02% active substance), and 24 hours later they are infected with a uredospore suspension of the fungus. After an incubation period of 48 hours (conditions: 95 to 100 percent relative humidity at 20°), the plants are placed in a greenhouse at 22°. 12 days after infection, the fungal attack is evaluated.

### b) Systemic action

5 days after planting, an aqueous spray mixture prepared from a wettable powder of the active ingredient (0.006% active substance, based on soil volume) is poured onto wheat plants. Care is taken that the spray mixture does not come into contact with the parts of the plants that are above ground. 48 hours later, the plants are infected with a uredospore suspension of the fungus. After an incubation period of 48 hours (conditions: 95 to 100 percent relative humidity at 20°), the plants are placed in a greenhouse at 22°. 12 days after infection, the fungal attack is evaluated.

### Example B-2: Activity against Phytophthora infestans on tomatoes

### a) Residual protective action

After cultivating for three weeks, tomato plants are sprayed to drip point with an aqueous spray mixture prepared from a wettable powder of the active ingredient (0.02% active substance), and 24 hours later they are infected with a sporangia suspension of the fungus. Evaluation of the fungal attack takes place 5 days after infection, during which time conditions of 90 to 100 percent relative humidity and a temperature of 20° are maintained.

### b) Systemic action

After cultivating for three weeks, an aqueous spray mixture prepared from a wettable powder of the active ingredient (0.006% active substance, based on soil volume) is poured onto tomato plants. Care is taken that the spray mixture does not come into contact with the parts of the plants that are above ground. 48 hours later, the plants are infected with a sporangia suspension of the fungus. Evaluation of the fungal attack takes place 5 days after infection, during which time conditions of 90 to 100 percent relative humidity and a temperature of 20° are maintained.

### Example B-3: Residual protective action against Cercospora arachidicola on peanuts

Peanut plants of 10 to 15 cm height are sprayed to drip point with an aqueous spray mixture prepared from a wettable powder of the active ingredient (0.02% active substance), and 48 hours later they are infected with a conidia suspension of the fungus. The plants are incubated for 72 hours at 21° and at high humidity, and then placed in a greenhouse until the typical leaf spots appear. Evaluation of the activity of the active substance is made 12 days after infection and is based on the number and size of leaf spots.

### Example B-4: Activity against Plasmopara viticola on grapevines

Vine seedlings at the 4 to 5 leaf stage are sprayed to drip point with an aqueous spray mixture prepared from a wettable powder of the active ingredient (0.02% active substance), and 24 hours later they are infected with a sporangia suspension of the fungus. Evaluation of the fungal attack takes place 6 days after infection, during which time conditions of 95 to 100 percent relative humidity and a temperature of 20° are maintained.

### Example B-5: Activity against Colletotrichum lagenarium on cucumbers

After cultivating for 2 weeks, cucumber plants are sprayed with an aqueous spray mixture prepared from a wettable powder of the active ingredient (concentration 0.002%). After 2 days, the plants are infected with a spore suspension (1.5x10⁵ spores/ml) of the fungus, and incubated for 36 hours at 23°C and at high humidity. Incubation then continues at normal humidity and at ca. 22°C. The fungal attack that has set in is evaluated 8 days after infection.

### Example B-6: Residual protective action against Venturia inaegualis on apples

Apple cuttings with new shoots 10 to 20 cm in length are sprayed to drip point with an aqueous spray mixture prepared from a wettable powder of the active ingredient (0.02% active substance), and 24 hours later they are infected with a conidia suspension of the fungus. The plants are incubated for 5 days at 90 to 100 percent relative humidity and placed in a greenhouse for a further 10 days at 20 to 24°. 12 days after infection, the fungal attack is evaluated.

### Example B-7: Activity against Erysiphe graminis on barley

### a) Residual protective action

Barley plants of approximately 8 cm height are sprayed to drip point with an aqueous spray mixture prepared from a wettable powder of the active ingredient (0.02% active substance), and 3 to 4 hours later they are dusted with conidia of the fungus. The infected plants are placed in a greenhouse at 22°. 12 days after infection, the fungal attack is evaluated.

### b) Systemic action

An aqueous spray mixture prepared from a wettable powder of the active ingredient (0.002% active substance, based on soil volume) is poured onto barley plants of approximately 8 cm height. Care is taken that the spray mixture does not come into contact with the parts of the plants that are above ground. 48 hours later, the plants are dusted with conidia of the fungus. The infected plants are placed in a greenhouse at 22°. 12 days after infection, the fungal attack is evaluated.

### Example B-8: Activity against Podosphaera leucotricha on apple shoots

Apple cuttings with new shoots of ca. 15 cm length are sprayed with a spray mixture (0.06% active substance). After 24 hours, the treated plants are infected with a conidia suspension of the fungus and placed in a plant-growth chamber at 70% relative humidity and at 20°C. 12 days after infection, the fungal attack is evaluated.

## Claims

1. A compound of formula I 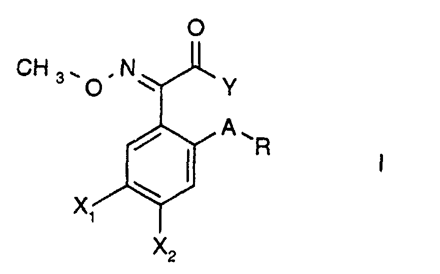 wherein:
A is a group CH₂O or CH₂ON=C(R₁);
X₁ and X₂ independently of one another, are C₁-C₄-alkyl;
Y is OH, O(C₁-C₄-alkyl), NH₂ or NHCH₃;
R₁ is C₁-C₄-alkyl, cyclopropyl, cyano, trifluoromethyl or C₁-C₄-alkoxy;
R is aryl, hetaryl or heterocyclyl, whereby the above-mentioned groups may be substituted by one or more identical or different substituents, selected from the group comprising halogen; C₁-C₆-alkyl; aryl which is optionally mono- to trisubstituted by identical or different substituents from C₁-C₄-alkyl, halogen, C₁-C₄-alkoxy, C₁-C₄-halogenalkyl or cyano;
C₁-C₆-alkoxy; halogen-C₁-C₆-alkoxy; aryloxy which is optionally mono- to trisubstituted by identical or different substituents from C₁-C₄-alkyl, halogen, C₁-C₄-alkoxy, C₁-C₄-halogenalkyl or cyano; halogen-C₁-C₆-alkyl; C₁-C₆-alkylthio; halogen-C₁-C₆-alkylthio; C₁-C₆-alkylsulfinyl; halogen-C₁-C₆-alkylsulfinyl; C₁-C₆-alkylsulfonyl;
halogen-C₁-C₆-alkylsulfonyl; C₂-C₆-alkenyl; C₂-C₆-alkenyloxy; C₂-C₆-alkynyl; C₃-C₆-alkynyloxy; halogen-C₂-C₆-alkenyl; halogen-C₂-C₆-alkenyloxy; halogen-C₂-C₆-alkynyl;
halogen-C₃-C₆-alkynyloxy; C₁-C₆-alkylcarbonyl; halogen-C₁-C₆-alkylcarbonyl;
C₁-C₆-alkoxycarbonyl; halogen-C₁-C₆-alkoxycarbonyl; C₁-C₆-alkylaminocarbonyl;
di-(C₁-C₆-alkyl)-aminocarbonyl, whereby the alkyl groups may be identical or different; C₁-C₆-alkylaminothiocarbonyl; di-(C₁-C₆-alkyl)-aminothicarbonyl, whereby the alkyl groups may be identical or different; C₁-C₆-alkylamino; di-(C₁-C₆-alkyl)-amino; NO₂; an unsubstituted C₁-C₄-alkylendioxy group or one which is mono- to tetrasubstituted by C₁-C₄-alkyl and/or halogen; CN; SF₅; OCN or C(=NOR₂ )-Z-R₃;
R₂ and R₃ independently of one another, are hydrogen or C₁-C₆-alkyl;
Z is a direct bond, O, S, NH or N(C₁-C₆-alkyl).

2. A compound according to claim 1, wherein R is phenyl which is unsubstituted or mono- to tri-substituted by identical or different substituents from halogen, C₁-C₆-alkyl, C₁-C₆-halogenalkyl, C₁-C₆-alkoxy, C₁-C₆-haloalkoxy, C₂-C₆-alkenyl, C₂-C₆-alkenyloxy, C₂-C₆-alkynyl, C₃-C₆-alkynyloxy, C₁-C₆-alkoxycarbonyl, cyano or OCN.

3. A compound according to claim 1, wherein R is phenyl which is substituted by C(=NOR₂)-Z-R₃, wherein R₂ and R₃ independently of one another signify hydrogen or C₁-C₄-alkyl and Z signifies a direct bond.

4. A compound according to claim 1, wherein R is pyridyl, pyrimidinyl, furyl, thienyl, oxazolyl, isoxazolyl, thiazolyl, isothiazolyl, imidazolyl, pyrazolyl or pyrazolinyl, which are unsubstituted or mono- to trisubstituted by identical or different substituents from halogen, cyano, nitro, aminocarbonyl, C₁-C₄-alkyl, C₁-C₄-halogenalkyl, C₁-C₄-alkylcarbonyl, C₁-C₄-alkylsulfonyl, C₁-C₆-alkylsulfoxyl, C₃-C₆-cycloalkyl, C₁-C₄-alkoxy, C₁-C₄-halogenalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₂-C₆-alkenyl, C(=NOR₂)-Z-R₃ or by aryl that is optionally mono- to trisubstituted by identical or different substituents from C₁-C₄-alkyl, halogen, C₁-C₄-alkoxy, C₁-C₄-halogenalkyl or cyano.

5. Process for the preparation of a compound of formula I **characterised by** the fact that
A) a compound of formula II 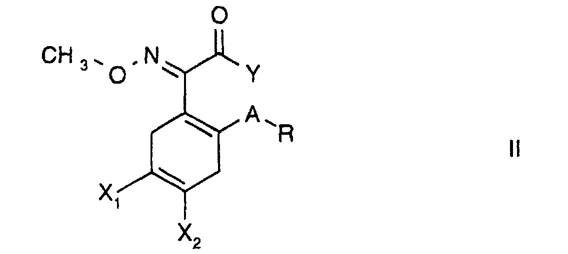 wherein A, R, X₁, X₂ and Y have the significances given for formula I, is reacted with an oxidation agent; or
B) in order to produce a compound of formula I, wherein Y is OH, NH₂ or NHCH₃ and A, R, X₁ and X₂ have the significances given for formula I, a compound of formula la 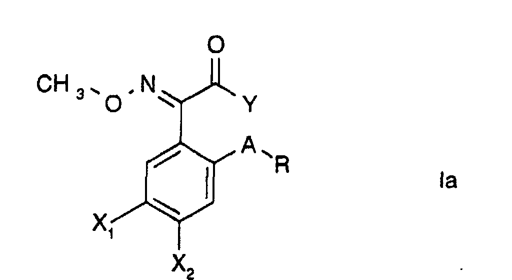 wherein Y signifies O(C₁-C₄ alkyl) and A, R, X₁ and X₂ have the significances given for formula I, is reacted with an aqueous acid or base, or with NH₃ or with NH₂CH₃; or
C) in order to produce a compound of formula I, wherein Y is (C₁-C₄-alkyl) or NHCH₃, a compound of formula III, 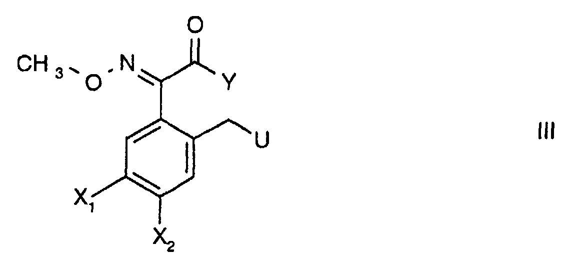 wherein U is a leaving group and the remaining substituents have the above-mentioned significances, is reacted with an alcohol of the general formula IV or with an oxime of the general formula V,
R-OH IV
R(R₁)C=NOH V
wherein R and R₁ have the significances given for formula I; or
D) a compound of formula VI, 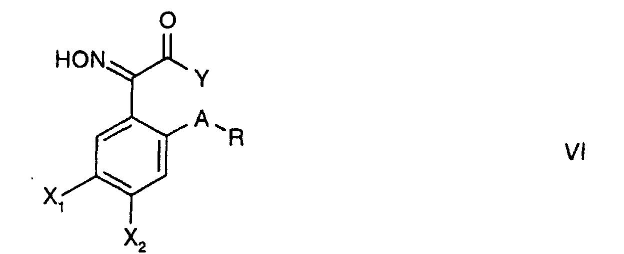 wherein the substituents have the significances given for formula I, is reacted with a methylation agent; or
G) in order to produce a compound of formula I, wherein A is the group CH₂ON=CR₁ and the remaining substituents have the significances given for formula I, a compound of formula IX, 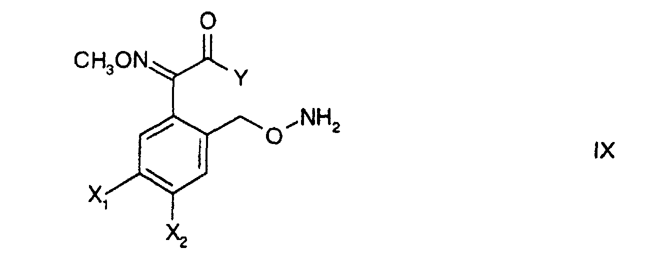 is reacted with a compound of formula R₁-CO-R, wherein the substituents have the significances given for formula I.

6. A compound selected from the group 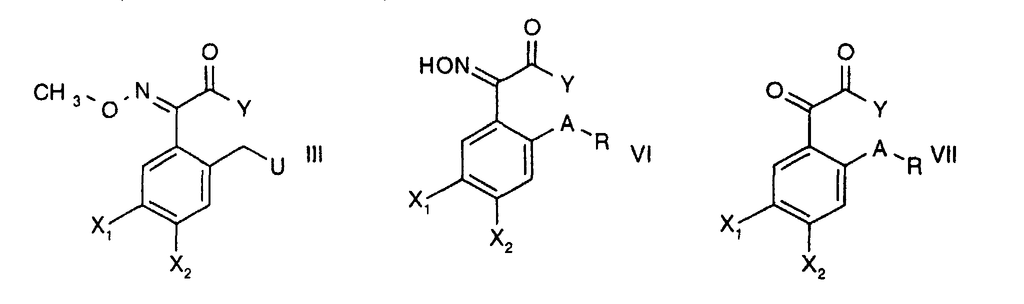 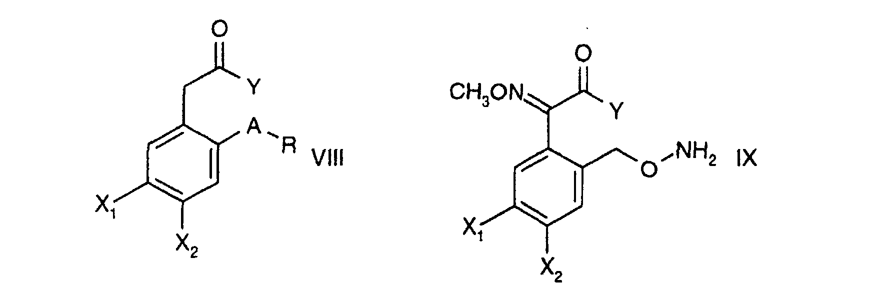 wherein the substituents have the significances given in claim 1 and U is a leaving group.

7. Agrochemical composition containing as active ingredient an effective quantity of a compound of formula I according to claim 1, together with an appropriate carrier.

8. Process for the protection of plants against harmful fungi, **characterised in that** a compound according to claim 1 is applied to the plants or to their locus.

## Patentansprüche

1. Verbindung der Formel I: 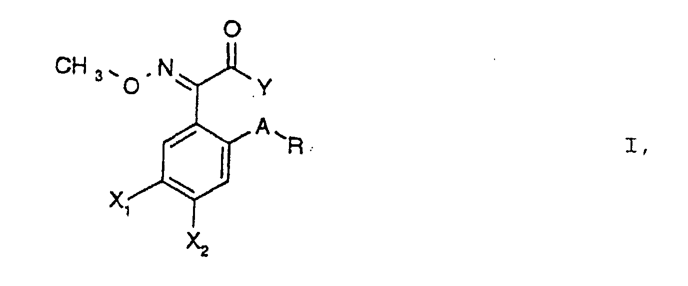 worin gilt:
A ist eine Gruppe CH₂O oder CH₂ON=C(R₁);
X₁ und X₂ sind, unabhängig von einander, C₁₋₄-Alkyl;
Y ist OH, O(C₁₋₄-Alkyl), NH₂ oder NHCH₃;
R₁ ist C₁₋₄-Alkyl, Cyclopropyl, Cyano, Trifluormethyl oder C₁₋₄-Alkoxy;
R ist Aryl, Hetaryl oder Heterocyclyl,
wobei die oben genannten Gruppen mit einem oder mehreren gleichen oder verschiedenen Substituenten substituiert sein können, ausgewählt aus der Gruppe, umfassend Halogen, C₁₋₆-Alkyl, Aryl, das gegebenenfalls mit gleichen der verschiedenen Substituenten aus C₁₋₄-Alkyl, Halogen, C₁₋₄-Alkoxy, C₁₋₄-Halogenalkyl oder aus Cyano mono- bis trisubstituiert ist, C₁₋₆-Alkoxy, Halogen-C₁₋₆-alkoxy, Aryloxy, das gegebenenfalls mit gleichen oder verschiedenen Substituenten aus C₁₋₄-Alkyl, Halogen, C₁₋₄-Alkoxy, C₁₋₄-Halogenalkyl oder aus Cyano monobis trisubstituiert ist, Halogen-C₁₋₆-alkyl, C₁₋₆-Alkylthio, Halogen-C₁₋₆-alkylthio, C₁₋₆Alkyl-sulfinyl, Halogen-C₁₋₆alkylsulfinyl, C₁₋₆-Alkylsulfonyl, Halogen-C₁₋₆alkylsulfonyl, C₂₋₆-Alkenyl, C₂₋₆-Alkenyloxy, C₂₋₆-Alkinyl, C₃₋₆Alkinyloxy, Halogen-C₂₋₆-alkenyl, Halogen-C₂₋₆alkenyloxy, Halogen-C₂₋₆-alkinyl, Halogen-C₃₋₆-alkinyloxy, C₁₋₆-Alkylcarbonyl, Halogen-C₁₋₆-alkylcarbonyl, C₁₋₆-Alkoxycarbonyl, Halogen-C₁₋₆-alkoxycarbonyl, C₁₋₆-Alkylaminocarbonyl, Di(C₁₋₆-alkyl)aminocarbonyl, worin die Alkylgruppen gleich oder verschieden sein können, C₁₋₆-Alkylaminothiocarbonyl, Di(C₁₋₆-Alkyl)aminothiocarbonyl, worin die Alkylgruppen gleich oder verschieden sein können, C₁₋₆-Alkylamino, Di(C₁₋₆-alkyl)amino, NO₂, eine unsubstituierte C₁₋₄-Alkylendioxygruppe oder eine, die mit C₁₋₄-Alkyl und/oder Halogen mono- bis tetrasubstituiert ist, CN, SF₅, OCN oder C(=NOR₂)-Z-R₃, worin R₂ und R₃, unabhängig von einander, Wasserstoff oder C₁₋₆-Alkyl und Z eine direkte Bindung, 0, S, NH oder N(C₁₋₆-Alkyl) sind.

2. Verbindung gemäß Anspruch 1, worin R Phenyl ist, das unsubstituiert oder mit gleichen oder verschiedenen Substituenten aus Halogen, C₁₋₆-Alkyl, C₁₋₆-Halogenalkyl, c₁₋₆-Alkoxy, C₁₋₆-Haloalkoxy, C₂₋₆-Alkenyl, C₂₋₆-Alkenyloxy, C₂₋₆-Alkinyl, C₃₋₆-Alkinyloxy, C₁₋₆-Alkoxycarbonyl, Cyano oder aus OCN mono- bis trisubstituiert ist.

3. Verbindung gemäß Anspruch 1, worin R Phenyl ist, das mit C(=NOR₂)-Z-R₃ substituiert ist, worin R₂ und R₃, unabhängig von einander, Wasserstoff oder C₁₋₄-Alkyl und Z eine direkte Bindung bedeuten.

4. Verbindung gemäß Anspruch 1, worin R Pyridyl, Pyrimidinyl, Furyl, Thienyl, Oxazolyl, Isoxazolyl, Thiazolyl, Isothiazolyl, Imidazolyl, Pyrazolyl oder Pyrazolinyl ist, die unsubstituiert oder mit gleichen oder verschiedenen Substituenten aus Halogen, Cyano, Nitro, Aminocarbonyl,
C₁₋₄-Alkyl, C₁₋₄-Halogenalkyl, C₁₋₄-Alkylcarbonyl, C₁₋₄-Alkylsulfonyl,
C₁₋₆-Alkylsulfoxyl, C₃₋₆-Cycloalkyl, C₁₋₄-Alkoxy, C₁₋₄-Halogenalkoxy,
C₁₋₆-Alkoxycarbonyl, C₁₋₆-Alkylthio, C₁₋₆-Alkylamino, Di-C₁₋₆alkylamino,
C₁₋₆-Alkylaminocarbonyl, Di-C₁₋₆-alkylaminocarbonyl, C₂₋₆-Alkenyl,
C(=NOR₂)-Z-R₃ oder aus Aryl mono- bis trisubstituiert ist,
das gegebenenfalls mit gleichen oder verschiedenen Substituenten aus
C₁₋₄-Alkyl, Halogen, C₁₋₄-Alkoxy, C₁₋₄-Halogenalkyl oder aus Cyano mono- bis trisubstituiert ist.

5. Verfahren zur Herstellung einer Verbindung der Formel I, **dadurch gekennzeichnet, dass**
A) eine Verbindung der Formel II: 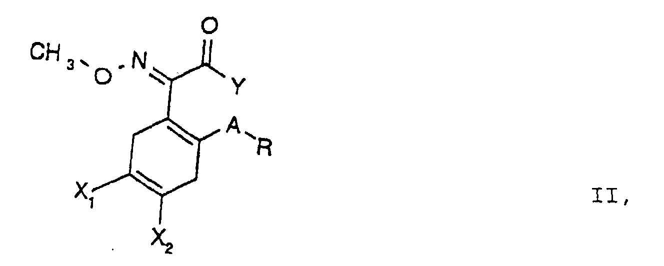 worin A, R, X₁, X₂ und Y die für Formel I angegebenen Bedeutungen haben, mit einem Oxidationsmittel zur Reaktion gebracht wird, oder dass
B) zur Erzeugung einer Verbindung der Formel I, worin Y OH, NH₂ oder NHCH₃ ist und A, R, X₁ und X₂ die für Formel I angegebenen Bedeutungen haben, eine Verbindung der Formel Ia: 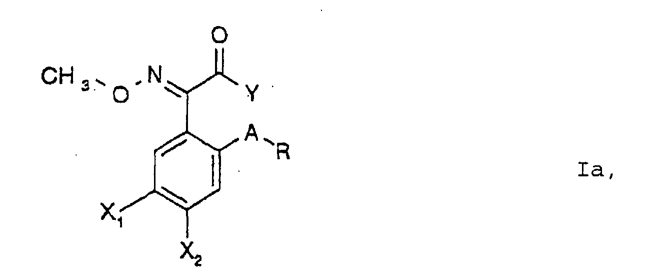 worin Y O(C₁₋₄-Alkyl) bedeutet und A, R, X₁ und X₂ die für Formel I angegebenen Bedeutungen haben, mit einer wässigen säure oder Base oder mit NH₃ oder mit NH₂CH₃ zur Reaktion gebracht wird, oder dass
C) zur Erzeugung einer Verbindung der Formel I, worin Y (C₁₋₄-Alkyl) oder NHCH₃ ist eine Verbindung der Formel III: 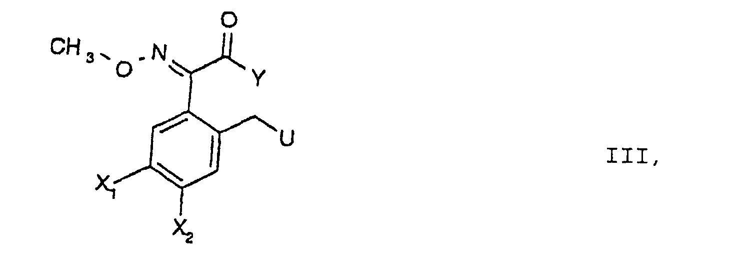 worin U eine Abgangsgruppe ist und die restlichen Substituenten die oben angegebenen Bedeutungen haben, mit einem Alkohol der allgemeinen Formel IV oder mit einem Oxim der allgemeinen Formel V zur Reaktion gebracht wird:
**R-OH IV,**
**R(R**_{**1**}**)C=NOH V,**
worin R und R₁ die für Formel I angegebenen Bedeutungen haben, oder dass
D) eine Verbindung der Formel VI: 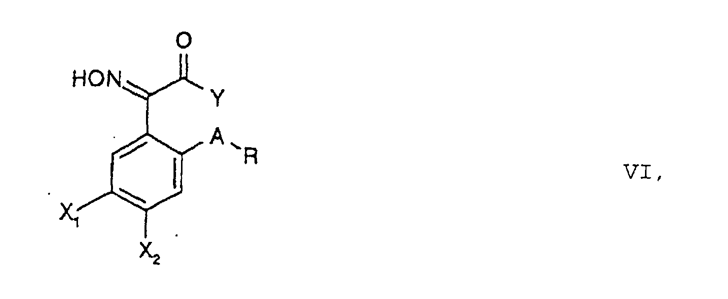 worin die Substituenten die für Formel I angegebenen Bedeutungen haben, mit einem Methylierungsmittel zur Reaktion gebracht wird, oder dass
G) zur Erzeugung einer Verbindung der Formel I, worin A die Gruppe CH₂ON=CR₁ ist und die restlichen Substituenten die für Formel I angegebenen Bedeutungen haben, eine Verbindung der Formel IX: 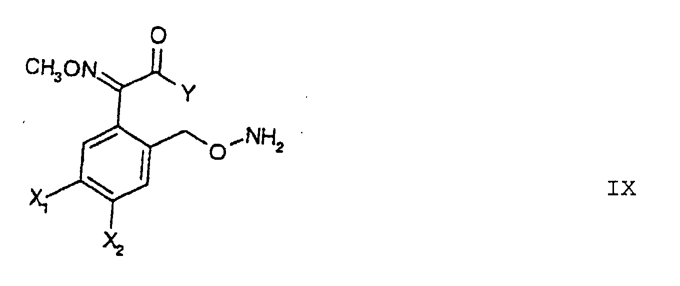 mit einer Verbindung der Formel R₁-CO-R zur Reaktion gebracht wird, worin die Substituenten die für Formel I angegebenen Bedeutungen haben.

6. Verbindung, ausgewählt aus der Gruppe: 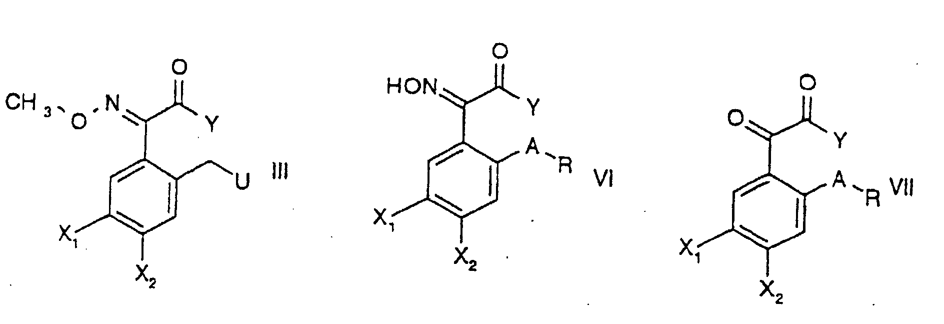 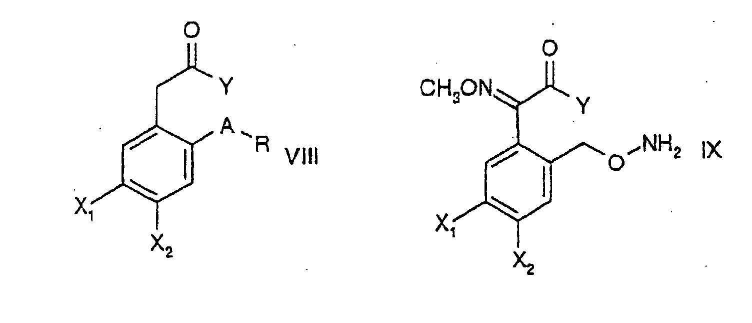 worin die Substituenten die in Anspruch 1 angegebenen Bedeutungen haben und U eine Abgangsgruppe ist.

7. Agrochemische Zusammensetzung, enthaltend als Wirkbestandteil eine wirksame Menge der Verbindung der Formel I gemäß Anspruch 1 zusammen mit einem geeigneten Träger.

8. Verfahren zum Schutz von Pflanzen vor schädlichen Fungi, **dadurch gekennzeichnet, dass**
eine Verbindung gemäß Anspruch 1 auf die Pflanzen oder deren Ort aufgebracht und angewandt wird.

## Revendications

1. Composé de formule I 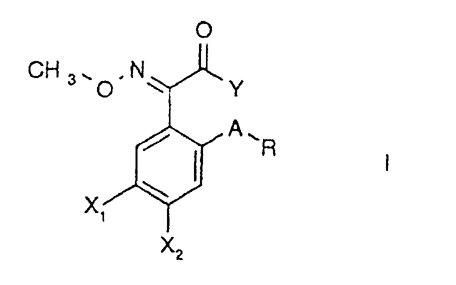 dans laquelle :
A représente un groupe CH₂O ou CH₂ON=C(R₁) ;
X₁ et X₂, indépendamment l'un de l'autre, représentent des groupes alkyle en C₁ à C₄ ;
Y représente un groupe OH, O(alkyle en C₁ à C₄), NH₂ ou NHCH₃ ;
R₁ représente un groupe alkyle en C₁ à C₄, cyclopropyle, cyano, trifluorométhyle ou alkoxy en C₁ à C₄ ;
R représente un groupe aryle, hétéroaryle ou hétérocyclyle, les groupes précités pouvant être substitués avec un ou plusieurs substituants identiques ou différents, choisis dans le groupe comprenant des substituants halogéno ; alkyle en C₁ à C₆ ; aryle qui est facultativement mono- à trisubstitué avec des substituants identiques ou différents choisis entre des substituants alkyle en C₁ à C₄, halogéno, alkoxy en C₁ à C₄, halogénalkyle en C₁ à C₄ et cyano ; alkoxy en C₁ à C₆ ; halogénalkoxy en C₁ à C₆ ; aryloxy qui est facultativement mono- à trisubstitué avec des substituants identiques ou différents choisis entre des substituants alkyle en C₁ à C₄, halogéno, alkoxy en C₁ à C₄, halogénalkyle en C₁ à C₄ et cyano ; halogénalkyle en C₁ à C₆ ; alkylthio en C₁ à C₆ ; halogénalkylthio en C₁ à C₆ ; alkylsulfinyle en C₁ à C₆ ; halogénalkylsulfinyle en C₁ à C₆ ; alkylsulfonyle en C₁ à C₆ ; halogénalkylsulfonyle en C₁ à C₆ ; alcényle en C₂ à C₆ ; alcényloxy en C₂ à C₆ ; alcynyle en C₂ à C₆ ; alcynyloxy en C₃ à C₆ ; halogénalcényle en C₂ à C₆ ; halogénalcényloxy en C₂ à C₆ ; halogénalcynyle en C₂ à C₆ ; halogénalcynyloxy en C₃ à C₆ ; (alkyle en C₁ à C₆)carbonyle ; halogéno(alkyle en C₁ à C₆)carbonyle ; (alkoxy en C₁ à C₆) carbonyle ; halogéno(alkoxy en C₁ à C₆)-carbonyle ; (alkyle en C₁ à C₆)aminocarbonyle ; di(alkyle en C₁ à C₆)aminocarbonyle, les groupes alkyle pouvant être identiques ou différents ; (alkyle en C₁ à C₆)aminothiocarbonyle ; di(alkyle en C₁ à C₆)aminothiocarbonyle, les groupes alkyle pouvant être identiques ou différents ; alkylamino en C₁ à C₆ ; di(alkyle en C₁ à C₆)amino ; NO₂ ; un groupe alkylènedioxy en C₁ à C₄ non substitué ou qui est mono- à tétrasubstitué avec des substituants alkyle en C₁ à C₄ et/ou halogéno ; CN ; SF₅ ; OCN ou C(=NOR₂)-Z-R₃ ;
R₂ et R₃, indépendamment l'un de l'autre, représentent des atomes d'hydrogène ou des groupes alkyle en C₁ à C₆ ;
Z représente une liaison directe, O, S, un groupe NH ou N(alkyle en C₁ à C₆).

2. Composé suivant la revendication 1, dans lequel R représente un groupe phényle qui est non substitué ou mono-à trisubstitué avec des substituants identiques ou différents choisis entre des substituants halogéno, alkyle en C₁ à C₆, halogénalkyle en C₁ à C₆, alkoxy en C₁ à C₆, halogénalkoxy en C₁ à C₆, alcényle en C₂ à C₆, alcényloxy en C₂ à C₆, alcynyle en C₂ à C₆, alcynyloxy en C₃ à C₆, aikoxycarbonyle en C₁ à C₆, cyano et OCN.

3. Composé suivant la revendication 1, dans lequel R représente un groupe phényle qui est substitué avec un substituant C(=NOR₂)-Z-R₃, dans lequel R₂ et R₃, indépendamment l'un de l'autre, représentent un atome d'hydrogène ou un groupe alkyle en C₁ à C₄ et Z représente une liaison directe.

4. Composé suivant la revendication 1, dans lequel R représente un groupe pyridyle, pyrimidinyle, furyle, thiényle, oxazolyle, isoxazolyle, thiazolyle, isothiazolyle, imidazolyle, pyrazolyle ou pyrazolinyle, qui est non substitué ou mono- à trisubstitué avec des substituants identiques ou différents choisis entre des substituants halogéno, cyano, nitro, aminocarbonyle, alkyle en C₁ à C₄, halogénalkyle en C₁ à C₄, (alkyle en C₁ à C₄)-carbonyle, alkylsulfonyle en C₁ à C₄, alkylsulfoxyle en C₁ à C₆, cycloalkyle en C₃ à C₆, alkoxy en C₁ à C₄, halogénalkoxy en C₁ à C₄, (alkoxy en C₁ à C₆) carbonyle, alkylthio en C₁ à C₆, alkylamino en C₁ à C₆, di(alkyle en C₁ à C₆)amino, (alkyle en C₁ à C₆)aminocarbonyle, di(alkyle en C₁ à C₆)-aminocarbonyle, alcényle en C₂ à C₆, C(=NOR₂)-Z-R₃, ou avec un groupe aryle qui est facultativement mono- à trisubstitué avec des substituants identiques ou différents choisis entre des substituants alkyle en C₁ à C₄, halogéno, alkoxy en C₁ à C₄, halogénalkyle en C₁ à C₄ et cyano.

5. Procédé pour la préparation d'un composé de formule I, **caractérisé en ce que**
A) un composé de formule II 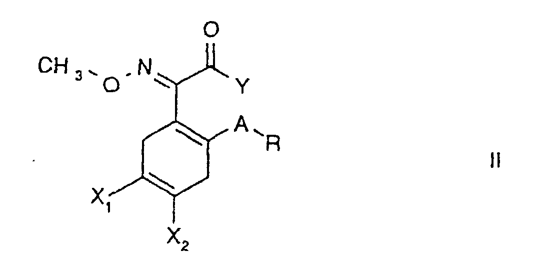 dans laquelle A, R, X₁, X₂ et Y répondent aux définitions mentionnées pour la formule I, est amené à réagir avec un agent d'oxydation ; ou
B) afin de produire un composé de formule I, dans laquelle Y représente un groupe OH, NH₂ ou NHCH₃ et A, R, X₁ et X₂ répondent aux définitions mentionnées pour la formule I, un composé de formule Ia 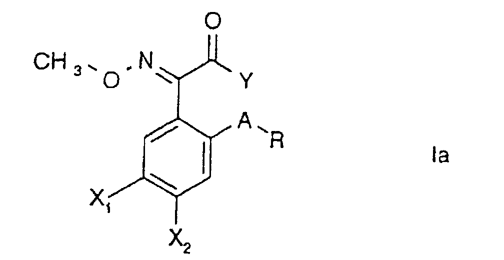 dans laquelle Y représente un groupe O(alkyle en C₁ à C₄) et A, R, X₁ et X₂ répondent aux définitions mentionnées pour la formule I, est amené à réagir avec une solution aqueuse d'un acide ou d'une base, ou avec NH₃ ou bien avec NH₂CH₃ ; ou
C) afin de produire un composé de formule I, dans laquelle Y représente un groupe alkyle en C₁ à C₄ ou NHCH₃, un composé de formule III 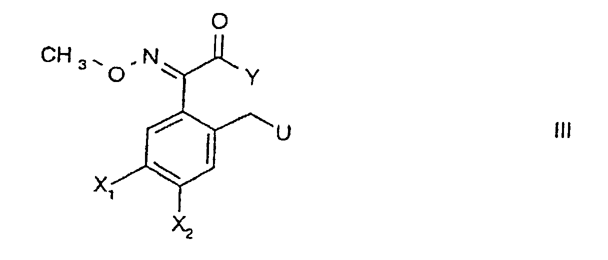 dans laquelle U représente un groupe partant et les substituants restants répondent aux définitions précitées, est amené à réagir avec un alcool de formule générale IV ou avec un oxime de formule générale V
R-OH IV
R(R₁)C=NOH V
dans laquelle R et R₁ répondent aux définitions mentionnées pour la formule I ; ou
D) un composé de formule VI 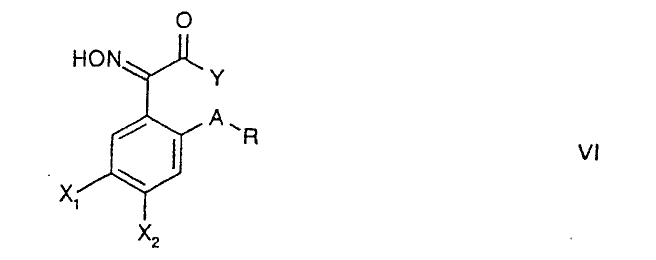 dans laquelle les substituants répondent aux définitions mentionnées pour la formule I, est amené à réagir avec un agent de méthylation ; ou
G) afin de produire un composé de formule I, dans laquelle A représente le groupe CH₂ON=CR₁ et les substituants restants répondent aux définitions mentionnées pour la formule I, un composé de formule IX 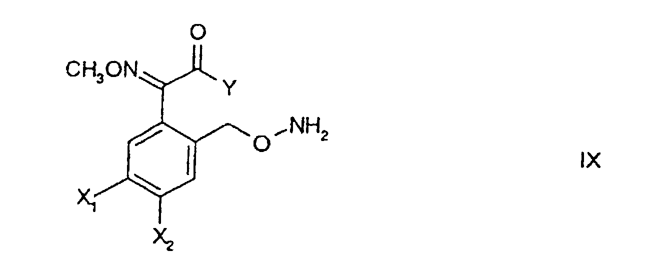 est amené à réagir avec un composé de formule R₁CO-R, dans laquelle les substituants répondent aux définitions mentionnées pour la formule I.

6. Composé choisi dans le groupe consistant en 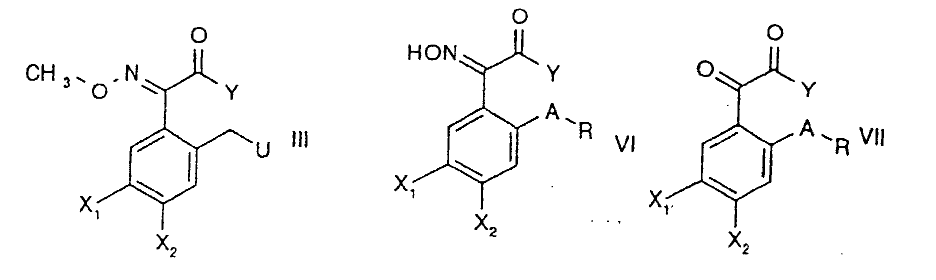 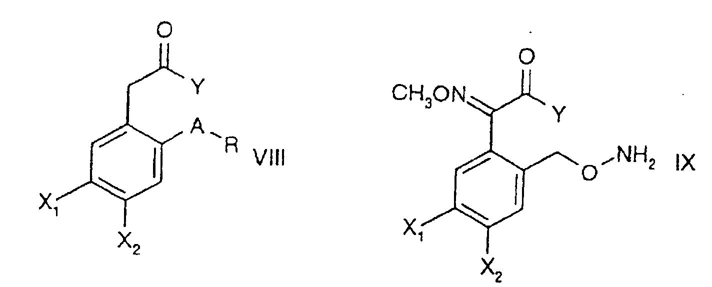 formules dans lesquelles les substituants répondent aux définitions mentionnées dans la revendication 1 et U représente un groupe partant.

7. Composition agrochimique contenant comme ingrédient actif une quantité efficace d'un composé de formule I suivant la revendication 1, conjointement avec un support approprié.

8. Procédé pour la protection de plantes contre des champignons nuisibles, **caractérisé en ce qu'**un composé suivant la revendication 1 est appliqué aux plantes ou au milieu dans lequel se trouvent ces plantes.
